# EUROPEAN PATENT SPECIFICATION

(11) **EP 2 022 474 B1**
(45) Date of publication and mention of the grant of the patent: **07.10.2015**
(21) Application number: 08013901.7
(22) Date of filing: 04.08.2008
(51) Int. Cl.: A61K 8/35, A61K 8/45, A61Q 5/02, A61Q 5/12

(54) **Conditioning composition for hair comprising optical brightener**
Zusammensetzung zur Haarkonditionierung mit einem optischen Aufheller
Composition de conditionnement pour cheveux comprenant un azurant optique

(30) Priority: 07.08.2007 EP 07015463
(43) Date of publication of application: 11.02.2009
(73) Proprietor: Kao Germany GmbH, 64297 Darmstadt (DE)
(72) Inventor: Hoffmann, Martin, 64673 Zwingenberg (DE); Molenda, Michael, 60487 Frankfurt (DE)
(74) Representative: Grit, Mustafa

(56) References cited:
- EP-A- 1 059 077
- EP-A- 1 598 051
- EP-A- 1 674 070
- WO-A-99/13823

## Description

The present invention is related to a conditioning composition for hair comprising optical brightener und ubichinone. The conditioning composition of the present invention can be in the form of a shampoo, cleansing - conditioning composition, or in the form of a conditioner used after washing hair with cleansing compositions.

Optical brightener comprising hair conditioning compositions are known in the literature. For example, WO 99/13823 discloses hair conditioning compositions comprising optical brighteners and a hair conditioning agent selected from silicones, cationic compounds, high melting point compounds, perfume compounds and water-soluble high molecular weight oily compounds. There is a need for further improvement in their performance with respect to the brightening and conditioning effect especially keeping healthy appearance of hair for a long time.

On the other hand deposition of the optical brighteners onto hair is found to be very weak from up until now known hair cosmetic compositions and consequently either low in effect or very high concentration of the actives are used for achieving the desired effects. Therefore, there is a great need for improvement in the deposition of optical brighteners onto hair to increase their effectiveness.

EP 1674070 A1 discloses compositions comprising optical brightener at a low pH range which further comprise at least one hydroxycarboxylic acid and/or dicarboxylic acid in order to increase deposition of optical brightener onto hair. It has been observed that hair conditioning properties of such compositions still need to be improved.

The objective of the current invention is to provide conditioning compositions for keratin fibers especially hair for effectively brightening hair and also conditioning hair so that hair looks healthy or healthier with excellent shine, manageability, combability and elasticity.

It has surprisingly been found out that a composition comprising at least one optical brightener and at least one ubichinone brightens hair excellently and also improves hair conditions in terms of combability, shine, elasticity and managabilty.

Accordingly the first object of the present invention is a conditioning composition for hair characterised in that it comprises at least one optical brightener and at least one ubichinone of the following formula 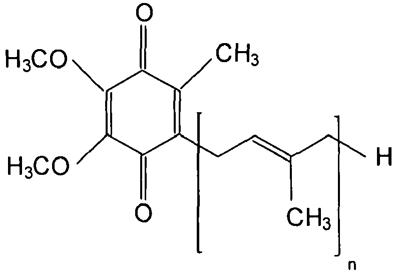 where n is a number between 1 and 10.

Another object of the present invention is the use of conditioning composition of above for brightening and conditioning hair.

Further object of the present invention is the use of the above composition for conditioning hair wherein the composition is not rinsed off from hair after application - so called leave-in application.

Still further object of the present invention is the method of conditioning hair wherein hair is treated with at least one composition as mentioned above and rinsed off from hair after a processing time of 30 sec. to 30 min.

Composition of the present invention comprises at least one optical brightener. Any optical brightener suitable for use in hair care area is in principal part of the present invention. Especially those mentioned in the PCT patent application WO 99/13823 are by reference included within the scope of the invention. The most preferred optical brightener is disodium distyrylbiphenyl disulfonate, known with the trade name Tinopal CBS-X from Ciba Geigy. Concentration of the optical brightener in the compositions of the present invention is typically in the range of 0.001% to 1%, by weight, preferably 0.01 to 0.75 and more preferably 0.01 to 0.5% by weight, calculated to total composition.

Compositions of the present invention comprise at least one ubichinone (also known as Coenzyme). It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

The compositions of the present invention can be either a conditioning -cleansing composition - shampoo - or a conditioning composition typically used after use of a cleansing compositions

The composition of the present invention comprises hair-conditioning agents in any type of composition. Conditioning agents can be selected from oily substances, non-ionic substances, cationic amphiphilic ingredients, cationic polymers or their mixtures. In the preferred embodiment of the present invention compositions comprise at least one conditioning compound selected from cationic surfactants, cationic polymers and silicone compounds especially silicone oils. Oily substances are selected from such as silicone oils, either volatile or nonvolatile, natural and synthetic oils. Among silicone oils those can be added to the compositions include dimethicone, dimethiconol, polydimethylsiloxane, DC fluid ranges from Dow Corning, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁CO(OCH₂CH₂)ₙOH

or

R₁ CO (O CH₂ CH₂)ₙ O OC R₂

where R₁ and R₂ are independent from each other saturated, unsaturated or branched or non-branched alkyl chain with 7 to 21 C atoms and n is typically 2 - 100.

In one of the preferred from of the present invention, conditioning compositions comprise at least one cationic polymer as conditioning agent. Suitable cationic polymers are those of best known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium-67, and Polyquaternium-70.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

It has further been found out that especially those of cationic cellulose type polymers known for example as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

Cationic polymers also include quaternized products of graft polymers from organopolysiloxanes and polyethyl oxazolines described in EP-A 524 612 and EP-A 640 643.

Conditioning compositions of the present invention can comprise additionally one or more cationic surfactant(s) as conditioner presented with the general formula 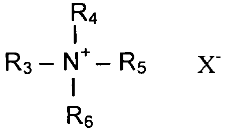 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has value of 1 - 4,
or

R₈ CO O (CH₂)ₙ

where R₈ is saturated or unsaturated, branched or non-branched alkyl chain with 7-21 C atoms and n has value of 1 - 4, and
R₄ is hydrogen or unsaturated or saturated, branched or non-branched alkyl chain with 1 - 4 C atoms or

R₇ CO NH (CH₂)ₙ

or

R₈ CO O (CH₂)ₙ

where R₇, R₈ and n are same as above.

R₅and R₆ are hydrogen or lower alkyl chain with 1 to 4 carbon atoms, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropyldimethylamine known with a trade name Tego Amid S18 from Degussa, and Lexamine S13 from Inolex.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants can be 0.01 - 10% by weight, preferably 0.01 - 7.5% by weight, more preferably 0.05 - 5% and most preferably 0.1 - 3% by weight calculated to the total composition. It should be noted that especially non-cleansing conditioning type of the products contain higher concentrations of the above mentioned concentrations of the cationic surfactants which at the same time if desired can be emulsifying agent. In cleansing and conditioning type of preparations, concentration of cationic surfactants is lower.

In a preferred for on the invention, conditioning compositions especially those used after cleansing hair comprise at least one cationic surfactant which is at the same time an emulsifier especially if it is a mono alkyl surfactant.

In another preferred from of the present invention, composition comprises at least one organic solvent and/or at least one UV filter and/or at least one direct dye as disclosed in detail below.

In another preferred form of the invention, it has been found out that in the presence of organic solvents, brightening and conditioning effects are further very much enhanced. Without being bound by any theory, it is thought that the accelerated/more pronounced effect is observed due to penetration enhancing effect of the organic solvents. Accordingly, conditioning composition can comprises organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyloxyethanol and polypropylene glycols. Concentration of organic solvents should not exceed 10% by weight, preferably in the range of 0.1 to 7.5%, more preferably 0.1 to 5% by weight and most preferably 0.1 to 3% by weight calculated to total composition.

Conditioning compositions of the present invention can be a cleansing composition (cleansing-conditioning composition). Cleansing conditioning compositions of the present invention comprise at least one surfactant selected from anionic, non-ionic and/or amphoteric or zwitterionic surfactants at a concentration range of 5 to 50%, preferably 5 to 40% and more preferably 5 to 30%, and most preferably 5 to 25% by weight, calculated to the total composition. In an embodiment of the present invention cleansing conditioning composition of the present invention, comprises at least one anionic, at least one nonionic surfactant. More preferably the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉-(C₂H₄O)ₙ-O-CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 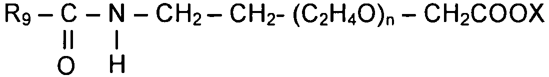 wherein R₉ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

An overview of the anionic surfactants used in liquid body cleansing compositions can furthermore be found in the monography of K. Schrader, "Grundlagen und Rezepturen der Kosmetika", 2nd Ed.(1989, Hüthig Buchverlag), pp. 595-600 and pp. 683 to 691.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in the shampoo compositions according to the invention are nonionic surfactants in admixture with anionic surfactants.

These are described in Schrader, I.c., on pages 600-601 and pp. 694-695. Especially suited are alkyl polyglucosides of the general formula

R₁₀-O-(R₁₁O)ₙ-Zₓ,

wherein R₁₀ is an alkyl group with 8 to 18 carbon atoms, R₁₁ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides which may be present in an amount from 0.25 % to 5 % by weight, calculated to the total composition.

Such amineoxides are state of the art, for example C₁₂-C₁₈- alkyl dimethyl amineoxides such as lauryl dimethyl amineoxide, C₁₂-C₁₈- alkyl amidopropyl or - ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

As further surfactant component, the compositions according to the invention can also contain amphoteric or zwitterionic surfactants, for example in an amount from about 0.5 % to about 15 %, preferably from about 1 % to about 10 %, by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 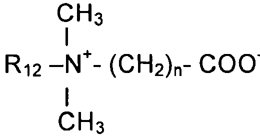 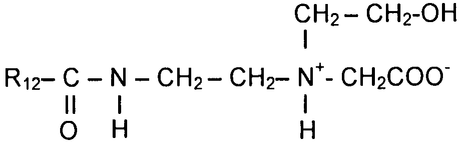 wherein R₁₂ is a C₈-C₁₈-alkyl group and n is 1 to 3;
sulfobetaines of the structure 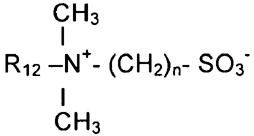 wherein R₁₂ and n are same as above;
and amidoalkyl betaines of the structure 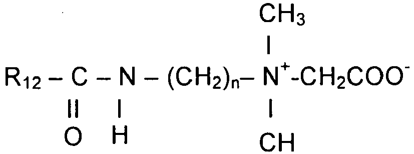 wherein R₁₂ and n are same as above.

Solubilizers may be added to the compositions, in particular cleansing compositions, especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1 % by weight, calculated to total composition.

Further conditioning additives are hair conditioning and/or styling polymers into either cleansing or conditioning type. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning composition of any type of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryloylethyl betaine and alkyl - methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Conditioning and cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. However, pearl-shiny appearance is achieved with those dispersed in cleansing color-enhancing compositions in crystalline form, i.e. so called pearlshine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kind of mixtures is available commercially.

Hair cleansing conditioning compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether and alkanes such as butane propane or their mixtures.

Conditioning compositions used after washing hair with a cleansing composition of the present invention can be in the form of emulsions, solutions, gels and dispersions. In the case that solutions and/or gels forms are preferred the appearance can be either with a transparent or opaque. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

The emulsion type of colouring conditioners comprise additionally at least one fatty alcohol of the following formula

R₁₃-OH

where R₁₃ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

The conditioning compositions of any type may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

The UV filters are those oil and water soluble ones for the purpose of protecting hair colour. In other words, anionic and nonionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebornane-2-one and the salts thereof, 3-(4'-methyl benzylidene)-DL-campher, and/or polysiliocne-15. The amount of the UV-absorber ranges typically from about 0.01 % to 2.5%, more preferably from 0.05 % to 1 % by weight, calculated to the total composition.

Natural plant extracts may be incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4th Ed..

The pH of the compositions according to the present invention is suitably between 2 and 8 and preferably in the range of 2.5 to 6.5, more preferably 3 to 5.5 and most preferably 3.5 to 5.

Furthermore as disclosed in EP 1674070 A1 in order further to enhance deposition of the optical brightener compositions of the present invention can comprise hydroxycarboxylic acid and/or dicarboxylic acid. The effects of both acids are observed below pH 4.5 optimally.

In principal pH of the compositions can be adjusted with any organic and/or inorganic acids or their mixture. Some of them to mention are phosphoric acid, hydrochloric acid as the inorganic ones and to the organic acids the well known citric acid and lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. It has further been observed that improved conditioning and bnrightening performace was observed when compositions comprise at the same time at least one hydroxycarboxylic and/or dicarboxylic acids.

Further in preferred embodiment of the present invention. Compositions comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuffs is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

The viscosity of the conditioning shampoo compositions according to the invention is in the range of 500 and about 20,000 mPa.s at 20°C, preferably 1,000 to 10,000, in particular 1,500 to 8,000 mPa.s at 20°C, measured with Höppler viscosimeter.

Viscosity of shampoo compositions can be adjusted with known viscosity enhancers. The preferred ones are glyceryl laurate, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

Viscosity of the non-cleansing conditioning composition may not be more than 50,000 mPa.s at 20°C measured with Brookfield Rheometer at a shear rate of 10 sec⁻¹.

It should especially be noted that the effects of the inventive compositions become more and more visible after repeated usage. Especially brightening and shine enhancing effects are very much pronounced after repeated usage.

The following examples are to illustrate the invention, but not to limit. The compositions according to the invention are prepared by mixing the individual components in water, whereby it is also possible to use pre-mixtures of various ingredients.

### Example 1

| | |
|---|---|
| Sodium lauryl ether sulfate | 11.0 (% by wt.) |
| Coco glucoside | 4.0 |
| Cocoamidopropyl betaine | 1.5 |
| Coenzyme Q10 | 0.05 |
| Cationic polymer (Polyquaternium-10) | 0.2 |
| Benzylalcohol | 0.25 |
| PEG-60-hydrogenated castor oil | 0.5 |
| PEG-18 Glyceryl cocoate/oleate | 2.0 |
| Disodium distyrylbiphenyl disulfonate | 0.25 |
| Citric acid | q.s. pH 5.5 |
| Perfume, preservative | q.s |
| Water | q.s. to 100.0 |

Hair washed with the above shampoo composition showed excellent shine and easily combable and improved elasticity and manageability. Exclusion of Ubichinone resulted in loss of effects. Furthermore, shine was very much reduced when Ubichinoe and optical brightener both excluded from the composition.

At the same time in the above composition 0.1 % Basic red 51 was dissolved. Excellent red shine was observed on dark blonde hair.

Similar results are observed with the following shampoo compositions.

### Example 2

| | |
|---|---|
| Sodium lauryl ether carboxylate (10EO) | 5.0 (% by wt.) |
| Coco glucoside | 5.0 |
| Cocoamidopropyl betaine | 5.0 |
| Cationic polymer (Polyquaternium-7) | 0.2 |
| Benzylalcohol | 0.5 |
| PEG-60-hydrogenated castor oil | 0.5 |
| PEG-18 Glyceryl cocoate/oleate | 1.0 |
| Disodium distyrylbiphenyl disulfonate | 0.25 |
| Ubichinone | 0.08 |
| Lactic acid | q.s. pH 5.0 |
| Perfume, preservative | q.s. |
| Water | ad 100.0 |

Further, into the above shampoo composition 0.1 % by weight Basic red 51, a cationic direct dye, was added. It was observed that hair washed with this shampoo had excellent red shine.

### Example 3

| | |
|---|---|
| Coco glucoside | 5.0 |
| Cocoamidopropyl betaine | 8.0 |
| Laureth-16 | 2.0 |
| Guar hydroxypropyltrimonium chloride | 0.5 |
| Benzophenone-3 | 0.2 |
| PEG-3 distearate | 0.8 |
| Coenzyme Q10 | 0.1 |
| PEG-18 Glyceryl cocoate/oleate | 0.80 |
| Disodium distyrylbiphenyl disulfonate | 0.1 |
| Malic acid | q.s. pH 4.0 |
| Perfume, preservative | q.s. |
| Water | ad 100.0 |

To the above composition, 0.1 % Basic orange 31 and 0.05% Basic red 76 was mixed. Hair washed with this shampoo had excellent warm blond shine.

### Example 4

| | |
|---|---|
| Coco glucoside | 5.0 |
| Cocoamidopropyl betaine | 6.0 |
| Laureth-16 | 4.0 |
| Cationic polymer (Polyquaternium-67) | 0.3 |
| Benzophenone-3 | 0.2 |
| Benzylalcohol | 0.5 |
| Ubichinone | 0.05 |
| Disodium distyrylbiphenyl disulfonate | 0.20 |
| Lactic acid | q.s. to pH 5.0 |
| Perfume, preservative | q.s. |
| Water | ad 100.0 |

The above composition is a very low viscosity composition, in any case a viscosity lower than 500 mPa.s measured at ambient temperature and with Höppler viscosimeter, confectioned into a pump-foamer as purchased from the company Air-Spray - Germany and showed excellent brightening and shine effect

Similarly and aerosol foam shampoo was prepared by confectioning the above composition at a weight ratio of 90/10 - composition/propellant- using propane-butane mixture as a propellant. The foam shampoo so obtained showed excellent cleansing and brightening and shine effects.

Additionally, inot the above shampoo 0.05% basic blue 99, and 0.005% basic red 51 was added. Excellent warm silver shine was observed on the washed gray hair. At the same time, excellent anti-yellow effect is observed on the freshly bleached hair.

### Example 5

### Hair treatment composition rinse-off

| | |
|---|---|
| Cetylstearylalcohol | 5.0 (% by weight) |
| Stearyltrimethylammoniumchlorid | 2.0 |
| Benzylalcohol | 2.5 |
| Disodium distyrylbiphenyl disulfonate | 0.1 |
| Ubichinone | 0.075 |
| Fragrance, preservative | q.s. |
| Lactic acid | q.s. pH 3.5 |
| Wasser | ad 100.0 |

Above composition is applied onto shampooed hair and processed for 5 min and rinsed off from hair. It was observed that wet hair is easily combable. In the dry state combability, manageability, elasticity and shine was very much improved.

In a further use, the above composition is used with the shampoo composition of Example 1. It was observed that especially the shine effect was very much higher compared to the use of te above composition with a conventional shampoo composition without ubichinone and optical brightener.

Accordingly, further object of the present invention is process for cleansing and conditioning hair characterized in that hair is washed with a cleansing composition comprising at least one foaming surfactant, at least one optical brightener and at least one ubichinone of the above formula and after rinsing off, a conditioning composition without cleansing effect comprising at least one optical brightener and at least one ubichinone of the above formula is applied and after a processing time of 1 to 30 min at ambient temperature rinsed off from hair.

Further object of the present invention is that in the above process after application of the conditioning composition without cleansing effect the composition is not rinsed off.

Still another object of the present invention is a kit for conditioning hair including a composition for cleansing comprising at least one foaming surfactant, at least one optical brightener and at least one ubichinone of the above formula and a composition for conditioning composition without cleansing effect comprising at least one optical brightener and at least one ubichinone of the above formula.

Furthermore into the above conditioner composition, hair direct dye Basic red 51 was included. After use on dark blonde hair am excellent red shine was observed on the hair.

### Example 6

### Foam conditioner

| | |
|---|---|
| Quaternium-80 | 0.2 (Gew.-%) |
| Polyquaternium-11 | 0.7 |
| PEG-60-hydrogenated ricinus oil | 0.5 |
| Ubichinone | 0.075 |
| Disodium distyrylbiphenyl disulfonate | 0.1 |
| Fragrance, preservative | q.s. |
| Lactic acid | q.s. to pH 3 |
| Wasser | ad 100.0 |

pH of the composition is adjusted to 3.4. The composition is suitable for leave-in and rinse off. In leave-in application, amount used is obviously less than in the case of a rinse of application. The composition is packed into an aerosol can with 90/10 ratio, by weight, liquid composition to propellant. As propellant propane, butane mixture is used.

Into the above composition 0.1 % Acid red 52 was added. It was possible to realize red shine onto medium blonde hair.

### Example 7

| | |
|---|---|
| Cetylstearylalcohol | 5.0 (% by weight) |
| Cetrimoniumchloride | 1.0 |
| Panthenol | 0,4 |
| Dimethicone | 0.75 |
| Hydroxypropyl Guar Hydroxypropyltrimonium | |
| Chloride | 1.0 |
| Disodium distyrylbiphenyl disulfonate | 0.2 |
| Ubichinone | 0.08 |
| Avocado extract | 0.5 |
| Fragrance, preservative | q.s. |
| Citric acid | q.s. to pH 3.0 |
| Wasser | ad 100.0 |

The above composition can be used as both leave-in and rinse off.

### Example 8

| | |
|---|---|
| Cetylstearylalcohol | 5.0 (% by weight) |
| Dioleoylethyldimethylammonium ethosulfate | 1.0 |
| Ceteareth 20 | 1.0 |
| Panthenol | 0,4 |
| Dimethicone | 0.75 |
| Hydroxypropyl Guar Hydroxypropyltrimonium | |
| Chloride | 1.0 |
| Disodium distyrylbiphenyl disulfonate | 0.2 |
| Ubichinone Q10 | 0.1 |
| Avocado extract | 0.5 |
| Fragrance, preservative | q.s. |
| Malic acid | q.s. to pH 3.5 |
| Wasser | ad 100.0 |

## Claims

1. Conditioning composition for hair **characterized in that**, it comprises at least one optical brightener and at least one compound according to formula 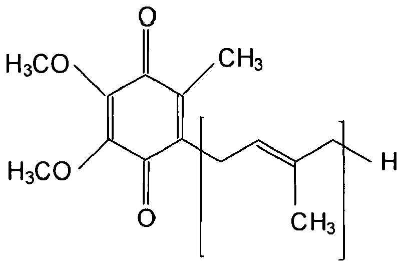 where n is a number between 1 and 10.

2. Composition according to claim 1 **characterized in that** the optical brightener is disodium distyrylbiphenyl disulfonate and present in the compositions at a concentration of 0.001 to 1% by weight, calculated to total composition.

3. Composition according to claims 1 and 2 **characterised in that** it comprises at least one Ubichinone at a concentration of 0.0001 to 1 % by weight calculated to total composition.

4. Composition according to any of the preceding claims **characterized in that** it comprises additionally at least one hair conditioning agent.

5. Composition according to claim 4 **characterized in that** conditioning agent is selected from cationic surfactants, cationic polymers and silicone compounds, preferably silicone oils and/or their mixtures.

6. Composition according to any of the preceding claims **characterized in that** compositions are cleansing and conditioning composition (shampoo) and comprising at least one surfactant selected from anionic, nonionic and amphoteric or zwitterionic surfactants at a concentration of 5 to 50% by weight calculated to the total composition.

7. Composition according to any of the preceding claims **characterized in that** it comprises at least one anionic surfactant and at least one non-ionic surfactant.

8. Composition according to claims 6 and 7 it comprises additionally at least one amphoteric surfactant.

9. Composition according to any of the preceding claims it comprises at least one organic solvent and/or at least one UV filter and/or at least one direct dye.

10. Composition according to any of the preceding claims **characterized in that** it has a pH in the range of 2.0 to 8.0.

11. Composition according to any of the preceding claims **characterized in that** it is an emulsion and comprises at least one fatty alcohol.

12. Use of composition according to any of the preceding claims for brightening and conditioning of hair.

13. Process for cleansing and conditioning hair **characterized in that** hair is washed with a cleansing composition comprising at least one foaming surfactant, at least one optical brightener and at least one ubichinone of the above formula and after rinsing off, a conditioning composition without cleansing effect comprising at least one optical brightener and at least one ubichinone of the above formula is applied and after a processing time of 30 sec to 30 min at ambient temperature rinsed off from hair.

14. Process according to claim 13 **characterized in that** after application of the conditioning composition without cleansing effect the composition is not rinsed off.

15. Kit for conditioning hair **characterized in that** it includes a composition for cleansing comprising at least one foaming surfactant, at least one optical brightener and at least one ubichinone of the above formula and a composition for conditioning composition without cleansing effect comprising at least one optical brightener and at least one ubichinone of the above formula.

## Patentansprüche

1. Konditionierzusammensetzung für Haar, **dadurch gekennzeichnet, dass** sie mindestens einen optischen Aufheller und mindestens eine Verbindung der Formel 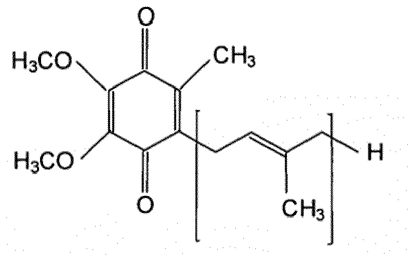 aufweist, wobei n eine Zahl von 1 bis 10 ist.

2. Zusammensetzung nach Anspruch 1, **dadurch gekennzeichnet, dass** es sich bei dem optischen Aufheller um Dinatriumdistyrylbiphenyldisulfonat handelt und dieser in einer Konzentration von 0,001 Gewichts-% bis 1 Gewichts %, bezogen auf die Gesamtzusammensetzung, in der Zusammensetzung vorliegt.

3. Zusammensetzung nach Anspruch 1 und 2, **dadurch gekennzeichnet, dass** sie mindestens ein Ubichinon in einer Konzentration von 0,0001 Gewichts-% bis 1 Gewichts % aufweist, bezogen auf die Gesamtzusammensetzung.

4. Zusammensetzung nach einem der vorhergehenden Ansprüche, **dadurch gekennzeichnet, dass** sie außerdem mindestens ein Haarkonditioniermittel aufweist.

5. Zusammensetzung nach Anspruch 4, **dadurch gekennzeichnet, dass** das Konditioniermittel aus kationischen Tensiden, kationischen Polymeren und Silikonverbindungen, vorzugsweise Silikonölen, und/oder deren Gemischen ausgewählt ist.

6. Zusammensetzung nach einem der vorhergehenden Ansprüche, **dadurch gekennzeichnet, dass** es sich bei der Zusammensetzung um eine Reinigungs- und Konditionierzusammensetzung (Shampoo) handelt, welche mindestens ein Tensid, ausgewählt aus anionischen, nichtionischen und amphoteren oder zwitterionischen Tensiden, in einer Konzentration von 5 Gewichts-% bis 50 Gewichts-% aufweist, bezogen auf die Gesamtzusammensetzung.

7. Zusammensetzung nach einem der vorhergehenden Ansprüche, **dadurch gekennzeichnet, dass** sie mindestens ein anionisches Tensid und mindestens ein nichtionisches Tensid aufweist.

8. Zusammensetzung nach Anspruch 6 und 7, sie weist außerdem mindestens ein amphoteres Tensid auf.

9. Zusammensetzung nach einem der vorhergehenden Ansprüche, sie weist mindestens ein organisches Lösungsmittel und/oder mindestens einen UV-Filter und/oder mindestens einen Direktfarbstoff auf.

10. Zusammensetzung nach einem der vorhergehenden Ansprüche, **dadurch gekennzeichnet, dass** sie einen pH-Wert im Bereich von 2,0 bis 8,0 aufweist.

11. Zusammensetzung nach einem der vorhergehenden Ansprüche, **dadurch gekennzeichnet, dass** sie eine Emulsion ist und mindestens einen Fettalkohol aufweist.

12. Verwendung der Zusammensetzung nach einem der vorhergehenden Ansprüche zum Aufhellen und Konditionieren von Haar.

13. Verfahren zum Reinigen und Konditionieren von Haar, **dadurch gekennzeichnet, dass** das Haar mit einer Reinigungszusammensetzung gewaschen wird, welche mindestens ein schäumendes Tensid, mindestens einen optischen Aufheller und mindestens ein Ubichinon der obigen Formel aufweist, und nach dem Ausspülen eine Konditionierzusammensetzung ohne Reinigungswirkung, welche mindestens einen optischen Aufheller und mindestens ein Ubichinon der obigen Formel aufweist, aufgetragen und nach einer Einwirkungszeit von 30 Sek. bis 30 Min. bei Umgebungstemperatur aus dem Haar ausgespült wird.

14. Verfahren nach Anspruch 13, **dadurch gekennzeichnet, dass** nach dem Auftragen der Konditionierzusammensetzung ohne Reinigungswirkung die Zusammensetzung nicht ausgespült wird.

15. Kit zum Konditionieren von Haar, **dadurch gekennzeichnet, dass** es eine Zusammensetzung zum Reinigen, welche mindestens ein schäumendes Tensid, mindestens einen optischen Aufheller und mindestens ein Ubichinon der obigen Formel aufweist, und eine Zusammensetzung zum Konditionieren ohne Reinigungswirkung umfasst, welche mindestens einen optischen Aufheller und mindestens ein Ubichinon der obigen Formel aufweist.

## Revendications

1. Composition de conditionnement capillaire **caractérisée en ce qu'**elle comprend au moins un agent éclaircissant et au moins un composé selon la formule 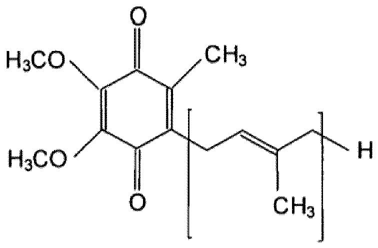 dans laquelle n est un nombre entre 1 et 10.

2. Composition selon la revendication 1, **caractérisée en ce que** l'agent éclaircissant est du disulfonate de distyrylbiphényle disodique et qu'il est présent dans les compositions à une concentration de 0,001 à 1 % en poids, calculée par rapport à la composition totale.

3. Composition selon les revendications 1 et 2, **caractérisée en ce qu'**elle comprend au moins une ubiquinone à une concentration de 0,0001 à 1 % en poids calculée par rapport à la composition totale.

4. Composition selon l'une quelconque des revendications précédentes, **caractérisée en ce qu'**elle comprend à titre complémentaire au moins un agent de conditionnement capillaire.

5. Composition selon la revendication 4, **caractérisée en ce que** l'agent de conditionnement est choisi parmi les tensioactifs cationiques, les polymères cationiques et les composés de silicone, de préférence les huiles silicones, et/ou leurs mélanges.

6. Composition selon l'une quelconque des revendications précédentes, **caractérisée en ce que** les compositions sont une composition nettoyante et une composition de conditionnement (shampooing), et comprenant au moins un tensioactif choisi parmi les tensioactifs anioniques, non ioniques et amphotères ou zwitterioniques à la concentration de 5 à 50 % en poids calculée par rapport à la composition totale.

7. Composition selon l'une quelconque des revendications précédentes, **caractérisée en ce qu'**elle comprend au moins un tensioactif anionique et au moins un tensioactif non ionique.

8. Composition selon les revendications 6 et 7, elle comprend à titre complémentaire au moins un tensioactif amphotère.

9. Composition selon l'une quelconque des revendications précédentes, elle comprend au moins un solvant organique et/ou au moins un filtre UV et/ou au moins un colorant direct.

10. Composition selon l'une quelconque des revendications précédentes, **caractérisée en ce qu'**elle a un PH dans l'intervalle de 2,0 à 8,0.

11. Composition selon l'une quelconque des revendications précédentes, **caractérisée en ce qu'**elle est une émulsion et comprend au moins un alcool gras.

12. Utilisation de la composition selon l'une quelconque des revendications précédentes pour l'éclaircissement et le conditionnement capillaire.

13. Procédé de nettoyage et de conditionnement capillaire, **caractérisé en ce que** le cheveu est lavé avec une composition nettoyante comprenant au moins un tensioactif moussant, au moins un agent d'éclaircissement et au moins une ubiquinone de la formule ci-dessus et, après rinçage, une composition de conditionnement sans effet nettoyant comprenant au moins un agent d'éclaircissement et au moins une ubiquinone de la formule ci-dessus est appliquée et après une durée de traitement entre 30 secondes et 30 minutes à température ambiante, la chevelure est rincée.

14. Procédé selon la revendication 13, **caractérisé en ce qu'**après l'application de la composition de conditionnement sans effet nettoyant, la composition n'est pas rincée.

15. Ensemble de conditionnement capillaire **caractérisé en ce qu'**il contient une composition nettoyante comprenant au moins un tensioactif moussant, au moins un agent d'éclaircissement et au moins une ubiquinone selon la formule ci-dessus et une composition pour une composition de conditionnement sans effet nettoyant comprenant au moins un agent d'éclaircissement et au moins une ubiquinone selon la formule ci-dessus.
